# EUROPÄISCHE PATENTANMELDUNG

(11) **EP 1 593 311 A1**
(43) Veröffentlichungstag der Anmeldung: **09.11.2005**
(21) Anmeldenummer: 04010947.2
(22) Anmeldetag: 07.05.2004
(51) Int. Cl.: A23K 1/00, A23K 1/165, A23K 1/16, A61K 9/16, A23L 1/00

(54) **Verfahren zum Herstellen von Lebensmitteln oder Futtermitteln**

(71) Anmelder: Amandus Kahl GmbH & Co. KG, 21465 Reinbek (DE)
(72) Erfinder: Sitzmann, Werner, Dr., 21075 Hamburg (DE); Graf von Reichenbach, Heinrich, Dr., 21465 Reinbek (DE); Behrmann, Joachim, 21465 Wentorf (DE)
(74) Vertreter: Glawe, Delfs, Moll

(57) **Zusammenfassung**

Verfahren zum Herstellen von Lebensmitteln oder Futtermitteln, denen Zusatzstoffe zugegeben werden, bei dem eine Vorbehandlung mit Erwärmung erfolgt und bei dem das Lebens- oder Futtermittel zu Pellets, Briketts, Granulat oder anderen stückigen Einheiten geformt wird, zeichnet sich dadurch aus, dass die Zugabe der Zusatzstoffe nach der Vorbehandlung und nach wenigstens teilweisem Abkühlen vor dem Formen zu stückigen Einheiten erfolgt.

## Beschreibung

Die Erfindung betrifft ein Verfahren zum Herstellen von Lebensmitteln oder Futtermitteln, denen Zusatzstoffe zugegeben werden, bei dem eine Vorbehandlung mit Erwärmung erfolgt und bei dem das Lebens- oder Futtermittel zu Pellets, Briketts, Granulat oder anderen stückigen Einheiten geformt wird.

Bei der Herstellung von Lebensmitteln und von Futtermitteln wie Mischfutter, Petfood und Fischfutter werden - insbesondere bei der Mischfutterproduktion - wichtige und hochwertige Zusatzstoffe, wie Enzyme, Vitamine, Leistungsförderer, Antioxidantien, Aroma- und appetitanregende Stoffe, Emulgatoren, Farbstoffe, Konservierungsstoffe, Stoffe zur Krankheitsverhütung bei den Nutztieren und andere Stoffe eingesetzt.

Die gängige Praxis heutzutage ist es, dass diese Zusatzstoffe, die nur in geringen Massenanteilen zum Einsatz kommen (Mikrokomponenten), zusammen mit einem Trägerstoff (Weizenkleie, Extraktionsschrote, tierische und pflanzliche Mehle) zu einer Vormischung vermischt und anschließend in einem Hauptmischer - der zentralen Maschine eines Mischfutterwerks - mit den massemäßig weitaus stärker vorhandenen sogenannten Makrokomponenten vermischt werden.

Dies kann kontinuierlich oder diskontinuierlich erfolgen.

Anschließend erfolgt üblicherweise die Konditionierung der gesamten auf diese Weise hergestellten Produktmischung in kontinuierlich betriebenen Maschinen und Apparaten, in denen thermische, hydrothermische, hydrothermisch-mechanische Verfahrensschritte realisiert werden, zum Zwecke der Produkthygienisierung sowie der Modifizierung von Inhaltsstoffen wie Stärke und Eiweiß.

Ein entscheidender Nachteil der konventionellen Vorgehensweise besteht darin, dass die zugefügten Zusatzstoffe überwiegend temperaturempfindlich und damit zerstörungsanfällig sind. Um im Endprodukt eine wirksame Restkonzentration dieser Wirkstoffe seitens des Mischfutterherstellers garantieren zu können, müssen diese Wirkstoffe daher entweder überdosiert zugegeben oder - beispielsweise durch entsprechende Schutzhüllen (Coatings) - vor den Auswirkungen höherer Temperaturen, wie sie in den nachgeschalteten Verfahrensschritten vorliegen müssen, um dort die gewünschten Veränderungen im Produkt überhaupt erreichen zu können, geschützt werden. Sowohl das Überdosieren als auch das Verwenden, teurerer gecoateter Wirkstoffe stellt einen erheblichen wirtschaftlichen Nachteil dar.

Um dieser unvermeidbaren thermischen Zerstörung von wichtigen Zusatzstoffen aus dem Wege zu gehen, hat man alternativ in den letzten Jahren den Weg des sogenannten "end-of-line"-Prozesses mit dem "post-pelleting-application" (PPA) eingeschlagen: Nach dem Pelletieren (und dem anschließenden Kühlen) als letztem Verfahrensschritt bei erhöhten Temperaturen werden Wirkstoffe auf die fertigen Pellets aufgesprüht. Da diese Wirkstoffe natürlich nur auf der Oberfläche der Pellets haften, zeigt sich in der Praxis, dass diese Wirkstoffe in nachfolgenden Prozessschritten durch mechanischen Abrieb (Transport, Lagerung) wieder von der Oberfläche abgetragen werden.

Die Aufgabe der Erfindung besteht in der Schaffung eines Verfahrens, mit dem die oben erwähnten wirtschaftlichen und prozesstechnischen Nachteile überwunden werden können und mit dem ein hoher Prozentsatz der verwendeten Zusatzstoffe in das Futtermittel gelangt.

Die erfindungsgemäße Lösung besteht darin, dass die Zugabe der Zusatzstoffe nach der Vorbehandlung und nach wenigstens teilweisem Abkühlen vor dem Formen zu stückigen Einheiten erfolgt.

Durch das Verfahren wird einerseits die Wärmebelastung durch den Vorgang des Vorkonditionierens für die Zusatzstoffe vermieden. Vor dem Zugeben der Zusatzstoffe lässt man das Material nach der Vorbehandlung soweit abkühlen oder kühlt es soweit, dass es eine niedrigere Temperatur erreicht, die den Zusatzstoffen nicht mehr oder in geringem Ausmaß schadet. Durch die anschließende Formung, insbesondere wenn diese in einem Extruder oder einer Pelletpresse erfolgt, findet zwar wieder eine Erwärmung statt. Es hat sich aber überraschenderweise gezeigt, dass diese erneute Erwärmung aufgrund der Vorbehandlung wesentlich geringer ist, als wenn keine vorbehandlung vorgenommen worden wäre. Es ist sozusagen ein Teil der Energie, der für die Formung aufgebracht werden muss und zur Erwärmung führt, bereits in der Vorbehandlung eingebracht. Die größte thermische Belastung, insbesondere auch hydrothermisch-mechanische Belastung in einem Expander ist bereits erfolgt, wenn die Wirkstoffe eingegeben werden. Trotzdem werden die Wirkstoffe aber hinreichend wirksam in das Material eingemischt, so dass, wenn die Formung der Produktmischung, die in einer Pelletpresse unter erhöhten Drücken und Temperaturen erfolgen kann, eine deutlich geringere Zerstörung der thermosensiblen Wirkstoffe auftritt, als dies bei konventionellen Verfahren der Fall ist.

Da der Großteil der Zusatzstoffe - im Gegensatz zum PPA-Verfahren - beim erfindungsgemäßen Verfahren sich im Inneren des Materialstroms befindet, der durch die Löcher der Matrize gedrückt, dort kompaktiert und zu Pellets geformt wird, ist dieser überwiegende Wirkstoffanteil gegen einen mechanischen Abtrag geschützt. Außerdem ist dieser "innere" Anteil auch gegen thermische Einflüsse geschützt, da bei der Pelletierung die jeweils höchsten Temperaturen an der Kontaktfläche zwischen Material und Bohrungsoberfläche und nicht im Inneren des späteren Pellets auftreten.

Erstaunlicherweise hat sich gezeigt, dass die Abbauraten von Phytase, einem sehr temperaturempfindlichen Enzym, bei der erfindungsgemäßen Vorgehensweise, selbst für den Fall der Anwendung eines Expanders als Beispiel für einen intensiven hydrothermisch-mechanischen Teilschritt, eine deutliche Reduzierung der Wirkstoffverluste gezeigt hat. Zu ähnlichen Ergebnisses kommt man bei der Zugabe von Vitamin K3 als Beispiel für die Zugabe temperaturempfindlicher Vitamine.

Bei einer vorteilhaften Ausführungsform wird eine thermische Vorbehandlung vorgenommen, also lediglich eine Erwärmung, z. B. durch indirekte Beheizung eines Behälters oder mit Heißluft, ggf. mit rezyklierter Heißluft.

Bei einer anderen vorteilhaften Ausführungsform wird eine hydrothermische Vorbehandlung vorgenommen. Das Ausgangsmaterial wird in diesem Falle mit Dampf aufgeheizt und befeuchtet.

Bei einer hydrothermisch-mechanischen Vorbehandlung erfolgt sowohl eine Befeuchtung und Erwärmung durch Dampf als auch eine mechanisch Behandlung. Ein Beispiel hierfür ist ein Expander. In einem Expander wird das zu behandelnde Material durch eine Förderschnecke gegen ein Austragsende gefördert, das eine verengte Austrittsöffnung hat. Das Material wird dabei intensiv mechanisch geknetet und bearbeitet. Diese mechanische Bearbeitung wird noch durch Schikanen, z. B. Stoppschrauben verstärkt, die im Weg angeordnet sind, den das Material zurücklegt. Das wesentliche beim Expander ist dabei dass die Erwärmung im wesentlichen oder sogar ausschließlich durch die starke mechanische Bearbeitung, also letztlich durch Reibung geschieht. Es kann zwar noch eine zusätzliche Erwärmung durch Dampf vorgenommen werden; erforderlich ist diese aber nicht. Das Material wird wie erwähnt in erster Line durch die mechanische Energieeinbringung - sehr stark erhitzt, vorzugsweise bis über 100°C. Verlässt das Material den Expander durch die Austrittsöffnung und wird es dort Atmosphärendruck ausgesetzt, so verdampft der unter mehr als 100°C stehende Wasseranteil spontan und bläht das Material auf. Diese Wirkung ist als Flash-Verdampfung bekannt.

Die Vorbehandlung könnte an sich auch in einem Extruder stattfinden, der sich vom Expander dadurch unterscheidet, dass das Material nicht durch eine im wesentlichen schlitzförmige Öffnung, sondern durch Bohrungen gepresst wird. Insbesondere im Falle des Extruders hat das Material dann schon eine ziemlich stabile stückige Form angenommen, so dass das Material möglicherweise erst wieder zerkleinert werden müsste, bevor die Zusatzstoffe eingemischt werden. Dies ist bei dem aus dem Expander austretenden Produkt weniger der Fall, obwohl auch hier eine Zerkleinerungseinrichtung vorgesehen sein kann. Als Zusatzstoffe kommen Vitamine, Enzyme oder medizinisch wirksame Zusatzstoffe in Frage. Diese werden nur in verhältnismäßig geringem Ausmaß hinzugefügt. Es können aber auch Zusatzstoffe hinzugefügt werden, die einen größeren Beitrag zum Nährwert liefern, wie Molkepulver, Milchpulver und andere Molkereiprodukte.

Wie erwähnt muss vor dem Zugeben der Zusatzstoffe eine Abkühlung erfolgen. Diese kann im Falle des Expanders oder Extruders die Flashverdampfung sein. Es ist aber auch eine Kühlung durch Kaltluftzuführung möglich.

Die Zusatzstoffe können in Pulverform oder aber in flüssiger Form zugegeben werden. Es versteht sich, dass es sich empfiehlt, nach dem Zugeben eine Mischung durchzuführen, um die Zusatzstoffe gleichmäßig im Material zu verteilen. Besonders vorteilhaft ist es, bei Einsatz eines Expanders diese Zusatzstoffe in das Expandat zu geben, da dann auf Grund der Struktur des Expandats eine gute Bindung der Produktteilströme zu erwarten ist.

Das Formen zu stückigen Einheiten kann in einer Pelletpresse erfolgen. Eine solche Pelletpresse ist sehr vorteilhaft zur Herstellung von Pellets, die von den Tieren gern als Nahrung angenommen werden. Das Formen kann aber auch in einer Brikettpresse erfolgen. Bei einer weiteren vorteilhaften Ausführungsform findet das Formen zu stückigen Einheiten in einem Extruder statt. Dabei kann die Vorbehandlung und das Formen zu stückigen Einheiten in einer einzigen Vorrichtung erfolgen, in der in einem rohrförmigen Gehäuse eine Press- und Förderschnecke angeordnet ist, die das Material unter Erwärmung durch eine schlitzförmige Öffnung, hinter der das Material unter Abkühlung expandiert, und die anschließend das Material durch eine Matrize durch Formgebung presst. Diese einteilige Vorrichtung besteht sozusagen aus einem Expander mit einem dahinter geschalteten Extruder. Die gesamte Vorrichtung arbeitet mit einer Welle. Wenn das Material die schlitzförmige Öffnung verlässt, tritt der Flash-Effekt auf, so dass sich das Material abkühlt. Zu diesem Zweck wird aus diesem Bereich Dampf abgelassen. Es kann dann eine weitere Kühlung durch Kaltluft erfolgen. Eine andere Möglichkeit ist, diesen Bereich hinter der schlitzförmigen Öffnung unter Unterdruck zu setzen, so dass das Material bis auf die Temperatur abkühlt, die dem Dampfdruck bei dem vorgegebenen Unterdruck entspricht.

Vorteilhafterweise ist der Durchtrittsquerschnitt der schlitzförmigen Öffnung veränderbar, um so den Durchsatz bzw. die Temperatur zu verändern. Eine solche einstückige Vorrichtung ist natürlich besonders zweckmäßig, kostengünstig und platzsparend.

Die Erfindung wird im folgenden anhand von vorteilhaften Ausführungsformen unter Bezugnahme auf die beigefügten Zeichnungen beschrieben. Es zeigen:
- Fig. 1: eine erste Ausführungsform einer Anlage zur Durchführung des erfindungsgemäßen Verfahrens;
- Fig. 2: eine zweite Ausführungsform mit einer einzigen Vorrichtung;
- Fig. 3: eine ähnliche Ausführungsform wie diejenige der Fig. 2;
- Fig. 4: den Verlust an Phytase bei Verfahren des Standes der Technik und der Erfindung; und
- Fig. 5: den Verlust an Vitamin K3 bei Verfahren des Standes der Technik und der Erfindung.

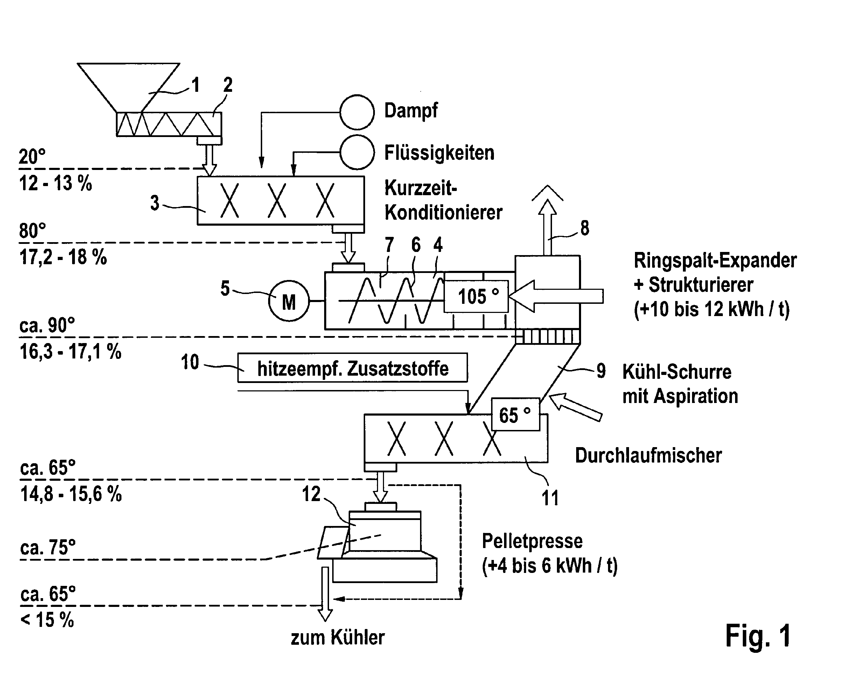

In Fig. 1 ist eine Anlage zur Durchführung des Verfahrens im Prinzip dargestellt, wobei jeweils die Temperaturen und die Produktfeuchten angegeben sind. Beim Expander und der Pelletpresse ist noch die elektrische Energie angegeben, die pro Tonne Futtermittel aufgewendet wird.

Das zu verarbeitende Material wird in einen Trichter 1 eingefüllt und durch eine Förderschnecke 2 in einen Kurzzeitkonditionierer 3 verbracht, in dem Dampf und Flüssigkeiten zugeführt werden können. Im Kurzzeitkonditionierer erfolgt eine Erwärmung und eine Erhöhung der Temperatur. Anschließend wird das Material in einen Ringspaltexpander 4 eingebracht, in dem es durch die Wirkung einer Förderschnecke und die dadurch eingebrachte mechanische Energie auf 105°C erhitzt wird. Der Antrieb erfolgt dabei durch einen Motor 5. Die Förder- bzw. Pressschnecke ist dabei bei 6 angeordnet. Sie besteht aus mehreren Teilen, die durch Schikanen in Form von Stoppschrauben 7 unterbrochen sind. Das durch die intensive mechanische Bearbeitung (zusätzlich kann noch Dampf zugegeben werden) auf 105°C erwärmte Material verlässt den engen Ringspalt expandiert dabei und wird durch einen Strukturierer zerkleinert. Dabei entweicht Dampf bei 8. Infolge der Flash-Verdampfung hat das Material noch eine Temperatur von 90°C und einen Feuchtigkeitsgehalt von 16,3 bis 17,1 %. In einer Kühlschurre 9 wird das Material weiter durch Kaltluft gekühlt, bis es eine Temperatur von 65° erreicht hat. Hier werden dann bei 10 die hitzeempfindlichen Zusatzstoffe hinzugeführt. Das Material wird anschließend in einem Durchlaufmischer 11 intensiv durchmischt, um die Zusatzstoffe mit dem im Expander vorbehandelten Material gründlich zu durchmischen. Das Material, das dann mit einer Temperatur von 65°C in die Pelletpresse 12 eintritt, wird in dieser dank der vorherigen Vorbehandlung nur noch wenig beansprucht und auf ca. 75°C erwärmt und verlässt die Pelletpresse 12 anschließend zum Kühler.

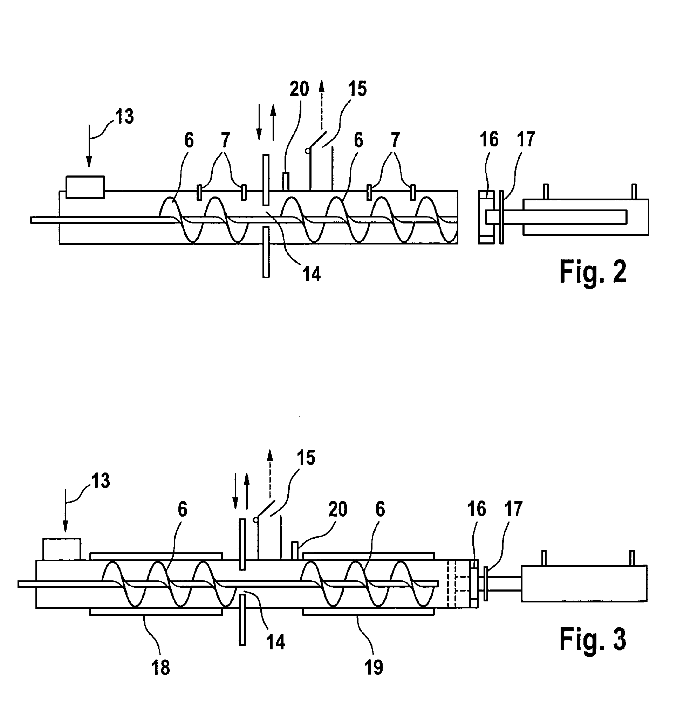

Bei der Ausführungsform der Fig. 2 ist ebenfalls eine Pressschnecke 6 vorgesehen, die ebenfalls aus mehreren Abschnitten besteht. Auch Schikanen in Form von Stoppschrauben 7 sind vorgesehen. Das von links in Richtung des Pfeiles 13 eingeführte Material wird durch die Pressschnecke 6 gegen einen Spalt 14 und durch diesen hindurchgedrückt, dessen Weite veränderbar wird. Durch die mechanische Bearbeitung durch die Schnecke 6 erfolgt dabei eine Erwärmung auf über 100°C, so dass das Material beim Durchtritt durch den Schlitz 14 sich spontan entspannt und abkühlt. Der Dampf, der hierbei frei wird, wird bei 15 abgeführt. Gegebenenfalls kann hier eine weitere Kühlung erreicht werden, indem dieser Teil der Vorrichtung mit Unterdruck beaufschlagt wird, um so die Verdampfungstemperatur abzusenken. Auch andere Arten der Abkühlung wären denkbar. Anschließend findet dann durch den rechten Teil der Pressschnecke 6 eine erneute Komprimierung statt, und das Material wird durch Matrizenbohrungen 16 hindurchgedrückt und verlässt diese in Form von Strängen, die mit einer Schneideinrichtung 17 in entsprechende Stücke unterteilt werden.

In Fig. 3 ist auch die Stelle 20 gezeigt, in der die Zusatzstoffe in flüssiger Form eingebracht werden, nämlich hinter dem Schlitz 14. Selbstverständlich wird auch bei der Ausführungsform der Fig. 2 an dieser Stelle Zusatzstoff eingeführt, was in Fig. 2 ebenfalls durch die Bezugsziffer 20 angedeutet ist.

In beiden in Fig. 2 und Fig. 3 gezeigten Ausführungsformen kann nach Bedarf im ersten Teil der Pressschnecke 6 vor dem Schlitz 14 durch eine erste Heiz- oder Kühleinrichtung 18 geheizt oder gekühlt werden. Ebenfalls nach Bedarf kann im zweiten Teil nach dem Schlitz 14 die Pressschnecke 6 durch eine zweite Heiz- oder Kühleinrichtung 19 geheizt oder gekühlt werden.

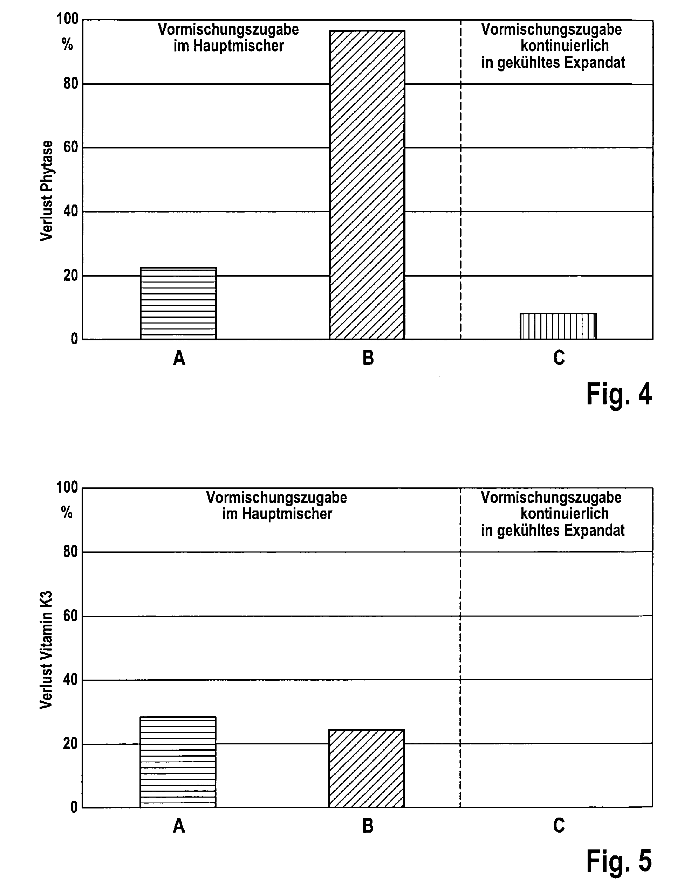

Fig. 4 zeigt den Verlust an Phytase. Zu diesem Zwecke wurde einerseits Phytase dem Futtermittel zugegeben, bevor es der Vorbehandlung und anschließenden Formung ausgesetzt wurde. Es zeigt sich dabei bei A ein Verlust von über 20 % wenn Mehl ohne hydrothermische Vorbehandlung pelletiert wird, bei B ein Verlust von fast 100 %, wenn eine hydrothermische Behandlung in einem Expander durchgeführt wird und anschließend pelletiert wird. Wird dagegen erfindungsgemäß die Phytase nach der Vorbehandlung und vor dem Pelletieren beigegeben, erhält man bei sonst gleichen Verhältnissen einen Verlust von weniger als 10 %.

In Fig. 5 ist der Verlust an Vitamin K3 bei den Fig. 4 entsprechenden Behandlungen gezeigt. Bei den vorbekannten zu mischenden vor der Vorbehandlung ergeben sich Verluste von über 20 %, beim erfindungsgemäßen Verfahren ein Verlust von praktisch 0 %.

## Patentansprüche

1. Verfahren zum Herstellen von Lebensmitteln oder Futtermitteln, denen Zusatzstoffe zugegeben werden, bei dem eine Vorbehandlung mit Erwärmung erfolgt und bei dem das Lebens- oder Futtermittel zu Pellets, Briketts, Granulat oder anderen stückigen Einheiten geformt wird, **dadurch gekennzeichnet, dass** die Zugabe der Zusatzstoffe nach der Vorbehandlung und nach wenigstens teilweisem Abkühlen vor dem Formen zu stückigen Einheiten erfolgt.

2. Verfahren nach Anspruch 1, **dadurch gekennzeichnet, dass** eine thermische Vorbehandlung vorgenommen wird.

3. Verfahren nach Anspruch 1, **dadurch gekennzeichnet, dass** eine hydrothermische Vorbehandlung vorgenommen wird.

4. Verfahren nach Anspruch 1, **dadurch gekennzeichnet, dass** eine hydrothermisch-mechanische Vorbehandlung vorgenommen wird.

5. Verfahren nach Anspruch 4, **dadurch gekennzeichnet, dass** eine Vorbehandlung in einem Expander oder Extruder vorgenommen wird.

6. Verfahren nach einem der Ansprüche 1 bis 5, **dadurch gekennzeichnet, dass** die Zusatzstoffe Vitamine aufweisen.

7. Verfahren nach einem der Ansprüche 1 bis 6, **dadurch gekennzeichnet, dass** die Zusatzstoffe Enzyme aufweisen.

8. Verfahren nach einem der Ansprüche 1 bis 7, **dadurch gekennzeichnet, dass** die Zusatzstoffe medizinisch wirksame Zusatzstoffe aufweisen.

9. Verfahren nach einem der Ansprüche 1 bis 8, **dadurch gekennzeichnet, dass** die Zusatzstoffe Molkepulver, Milchpulver oder andere Molkereiprodukte aufweisen

10. Verfahren nach einem der Ansprüche 4 bis 9, **dadurch gekennzeichnet, dass** die Kühlung durch Flashverdampfung am Ende der hydrothermisch-mechanischen Behandlung erfolgt.

11. Verfahren nach einem der Ansprüche 1 bis 10, **dadurch gekennzeichnet, dass** die Kühlung durch Kaltluftzuführung erfolgt.

12. Verfahren nach einem der Ansprüche 1 bis 11, **dadurch gekennzeichnet, dass** die Zusatzstoffe in Pulverform zugegeben werden.

13. Verfahren nach einem der Ansprüche 1 bis 11, **dadurch gekennzeichnet, dass** die Zusatzstoffe in flüssiger Form zugegeben werden.

14. Verfahren nach einem der Ansprüche 1 bis 13, **dadurch gekennzeichnet dass** das Formen zu stückigen Einheiten in einer Pelletpresse erfolgt.

15. Verfahren nach einem der Ansprüche 1 bis 13, **dadurch gekennzeichnet, dass** das Formen zu stückigen Einheiten in einer Brikettpresse erfolgt.

16. Verfahren nach einem der Ansprüche 1 bis 13, **dadurch gekennzeichnet, dass** das Formen zu stückigen Einheiten in einem Extruder erfolgt.

17. Verfahren nach Anspruch 16, **dadurch gekennzeichnet, dass** die Vorbehandlung und das Formen zu stückigen Einheiten in einer einzigen Vorrichtung erfolgt, in der in einem rohrförmigen Gehäuse eine Press- und Förderschnecke angeordnet ist, die das Material unter Erwärmung durch eine schlitzförmige Öffnung presst, hinter der das Material unter Abkühlung expandiert, und die anschließend das Material durch eine Matrize zur Formgebung presst.

18. Verfahren nach Anspruch 17, **dadurch gekennzeichnet, dass** der Durchtrittsquerschnitt der schlitzförmigen Öffnung veränderbar ist.

19. Verfahren nach Anspruch 17 oder 18, **dadurch gekennzeichnet, dass** hinter der schlitzförmige Öffnung Dampf abgelassen wird.

20. Verfahren nach einem der Ansprüche 17 bis 19, **dadurch gekennzeichnet, dass** hinter der schlitzförmigen Öffnung ein Unterdruck angelegt wird.
